# EUROPEAN PATENT SPECIFICATION

(11) **EP 3 144 383 B1**
(45) Date of publication and mention of the grant of the patent: **10.06.2020**
(21) Application number: 15792954.8
(22) Date of filing: 24.04.2015
(51) Int. Cl.: C12N 1/21, C12N 15/77, C12P 13/08

(54) **CORYNEBACTERIUM GENUS MICROORGANISM FOR PRODUCING L-LYSINE AND METHOD FOR PRODUCING L-LYSINE BY USING SAME**
MIKROORGANISMUS DER GATTUNG CORYNEBAKTERIUM MIT VERBESSERTER FÄHIGKEIT ZUR PRODUKTION VON L-LYSIN UND VERFAHREN ZUR HERSTELLUNG VON L-LYSIN DAMIT
MICRO-ORGANISME DU GENRE CORYNEBACTERIUM POUR PRODUIRE UNE L-LYSINE ET PROCÉDÉ DE PRODUCTION DE L-LYSINE AU MOYEN DU MICRO-ORGANISME

(30) Priority: 14.05.2014 KR 20140057966
(43) Date of publication of application: 22.03.2017
(73) Proprietor: CJ Cheiljedang Corporation, Seoul 100-400 (KR)
(72) Inventor: LEE, Seung Bin, Goyang-si Gyeonggi-do 411-705 (KR); KIM, Hyung Joon, Seoul 152-795 (KR); HUR, Lan, Seoul 134-817 (KR); MOON, Jun Ok, Seoul 158-090 (KR)
(74) Representative: Potter Clarkson
(86) International application number: PCT/KR2015/004092
(87) International publication number: WO 2015/174655

(56) References cited:
- WO-A1-2008/082180
- KR-A- 20070 060 798
- KR-B1- 100 789 271
- KR-B1- 101 285 945
- US-A1- 2003 003 548
- S. KLAFFL ET AL: "Genetic and Functional Analysis of the Soluble Oxaloacetate Decarboxylase from Corynebacterium glutamicum", JOURNAL OF BACTERIOLOGY, vol. 192, no. 10, 16 March 2010 (2010-03-16), pages 2604-2612, XP055189777, ISSN: 0021-9193, DOI: 10.1128/JB.01678-09
- TINGTING RAN ET AL: "Expression, purification, crystallization and preliminary crystallographic analysis of Cg1458: a novel oxaloacetate decarboxylase from Corynebacterium glutamicum", ACTA CRYSTALLOGRAPHICA SECTION F STRUCTURAL BIOLOGY AND CRYSTALLIZATION COMMUNICATIONS, vol. 67, no. 8, 1 August 2011 (2011-08-01) , pages 968-970, XP055407456, DOI: 10.1107/S1744309111023220
- DATABASE NCBI [Online] 4DBF_A, 07 December 2012 'CHAIN A, CRYSTAL STRCUTURES OF CG1458', XP055360702 Retrieved from NCBI Database accession no. 4DBF_A

## Description

### TECHNICAL FIELD

The present application relates to a microorganism of the genus *Corynebacterium* producing L-lysine and a method for producing L-lysine using the same.

### BACKGROUND ART

L-lysine is biosynthesized from a precursor oxaloacetate via a lysine biosynthetic pathway, and oxaloacetate is degraded to pyruvate and carbon dioxide by oxaloacetate decarboxylase (odx).

Previously, Simon Klaffl and Bernhard J. Eikmanns simply demonstrated that a gene cg1458 encodes oxaloacetate decarboxylase by confirming that overexpression of the intrinsic gene cg1458 in *Corynebacterium glutamicum* increases oxaloacetate decarboxylase activity and also decreases L-lysine production, and the oxaloacetate decarboxylase activity is attenuated by inactivation of the gene, but they did not demonstrate changes in the L-lysine production by inactivation of cg1458 (Klaffl S, Eikmanns BJ, et al., J. Bacteriol., May 2010, 192(10), p.2604-12). However, the present inventors predicted that when activity of the intrinsic oxaloacetate decarboxylase is inhibited in an L-lysine-producing *Corynebacterium* strain, the supply of oxaloacetate for the L-lysine biosynthetic pathway is increased without consumption for oxaloacetate in other pathways, and they inactivated the oxaloacetate decarboxylase gene in different classes of L-lysine-producing strains. As a result, the present inventors found that inactivation of the oxaloacetate decarboxylase gene results in improving the L-lysine-producing ability of the strain, thereby completing the present application.

### DETAILED DESCRIPTION OF THE INVENTION

### TECHNICAL PROBLEM

The invention is as defined in the accompanying claims. Accordingly, an object of the present application is to provide a microorganism of the genus *Corynebacterium* producing L-lysine.

Another object of the present application is to provide a method for producing L-lysine using the microorganism of the genus *Corynebacterium.*

### TECHNICAL SOLUTION

In order to achieve the above objects, an aspect of the present application provides a microorganism of the genus *Corynebacterium* producing L-lysine, in which an oxaloacetate decarboxylase activity is inactivated.

Further, another aspect of the present application provides a method for producing L-lysine by culturing the microorganism of the genus *Corynebacterium.*

### ADVANTAGEOUS EFFECTS OF THE INVENTION

The present application provides a recombinant microorganism of the genus *Corynebacterium* which has enhanced L-lysine production by inactivating an oxaloacetate decarboxylase gene in an L-lysine-producing *Corynebacterium* strain. The recombinant microorganism of the genus *Corynebacterium* is able to produce L-lysine with a high yield, thereby having industrially useful applications.

### DESCRIPTION OF THE DRAWINGS

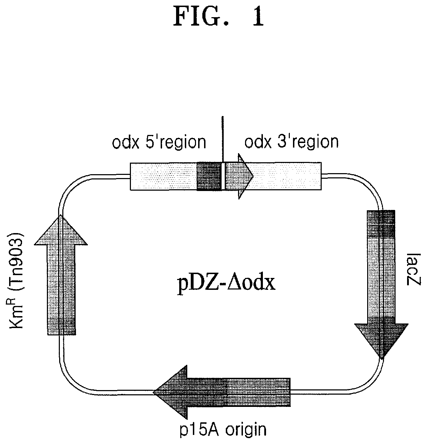

FIG. 1 shows a pDZ-Δodx vector for deleting an odx gene in the chromosome of *Corynebacterium.*

### MODE OF THE INVENTION

Hereinafter, the present application will be described in detail.

An aspect of the present application provides a microorganism of the genus *Corynebacterium* which has enhanced L-lysine production by inactivating an oxaloacetate decarboxylase activity, wherein oxaloacetate decarboxylase having an amino acid sequence set forth in SEQ ID NO: 1 is inactivated, wherein the microorganism has an enhanced L-lysine productivity compared to an unmodified microorganism, and wherein the microorganism comprises the *aspB* gene, *lysC* gene, *asd* gene, *dapA* gene, *dapB* gene and *lysA* gene.

Oxaloacetate decarboxylase (odx) is an enzyme that catalyzes degradation of oxaloacetate to pyruvate and carbon dioxide, and an enzyme derived from any microorganism may be used, as long as the enzyme has the above activity. Specifically, an enzyme derived from a Coryneform microorganism, and more specifically, an enzyme derived from *Corynebacterium glutamicum* may be used, but is not limited thereto.

The oxaloacetate decarboxylase of the present application has the amino acid sequence of SEQ ID NO: 1, and may be encoded by the odx gene having a nucleotide sequence of SEQ ID NO: 2. Any variant of the above nucleotide sequence encoding the same amino acid sequence, which results from genetic code degeneracy, may also be included in the present application.

The term 'homology', as used herein, refers to a degree of matching with a given amino acid sequence or nucleotide sequence, and the homology may be expressed as a percentage. A homology sequence having an activity which is identical or similar to the given amino acid sequence or nucleotide sequence is expressed as "% homology". The homology sequence may be determined by, for example, algorithm BLAST [see Karlin and Altschul, Pro. Natl. Acad. Sci. USA, 90, 5873(1993)] or FASTA by Pearson [see Methods Enzymol., 183, 63(1990)]. Based on this algorithm, a program called BLASTN or BLASTX has been developed [see http://www.ncbi.nlm.nih.gov]. The homology sequence may be also identified by comparing the sequences in a Southern hybridization experiment under stringent conditions as defined, and defining appropriate hybridization conditions are within the skill of the art (see e.g., Sambrook et al., 1989, infra.), and may be determined by a method well known to those skilled in the art.

The term 'inactivation', as used herein, means that the activity is further attenuated or eliminated, compared to a protein activity that occurs in a natural state or an unmodified state of an original microorganism, and the inactivation may be performed by any inactivation method known in the art. In the present application, inactivation of oxaloacetate decarboxylase means that the intrinsic odx gene is expressed at a low level or is not expressed, compared to that of a parent strain or an unmodified strain, or the activity is eliminated or decreased, even though the gene is expressed.

Specifically, the intrinsic activity of oxaloacetate decarboxylase may be inactivated by introducing a recombinant vector including a part of the gene encoding oxaloacetate decarboxylase into a microorganism to delete or mutate the intrinsic oxaloacetate decarboxylase gene, the expression of the protein may be attenuated by modifying a nucleotide sequence of a promoter region or 5'-UTR region of the gene, or the activity of the protein may be attenuated by introducing a mutation into an ORF region of the corresponding gene. Further, insertion of the gene into the chromosome may be performed by any method known in the art.

The term 'intrinsic activity', as used herein, refers to a protein activity of an original microorganism in a natural state or an unmodified state.

More specifically, the inactivation may be caused by site-specific gene disruption, but is not limited thereto.

The term 'recombinant vector', as used herein, refers to a DNA construct including a polynucleotide sequence of a polynucleotide encoding a target protein, which is operably linked to a suitable regulatory sequence so that the target protein may be expressed in an appropriate host. The regulatory sequence may include a promoter capable of initiating transcription, any operator sequence for regulating the transcription, a sequence encoding a suitable mRNA ribosome-binding site, and a sequence regulating termination of transcription and translation. The vector, after being transformed into a suitable host cell, may be replicated or function irrespective of the host genome, or may be integrated into the genome itself. The vector used in the present application is not particularly limited, as long as the vector is replicable in the host, and any vector known in the art may be used.

The term 'transformation', as used herein, means that a gene is introduced into a host cell, thereby enabling the expression of the gene in the host cell. The transformed gene may be located inside or outside the chromosome of the host cell, as long as it may be expressed in the host cell. The gene may be introduced in any form, as long as it may be introduced into the host cell and expressed therein.

As a parent cell into which the recombinant vector is introduced, any microorganism of the genus *Corynebacterium* may be used, as long as the microorganism is able to produce L-lysine. Specifically, *Corynebacterium glutamicum* may be used.

In a specific embodiment of the present application, a recombinant vector was constructed by inserting a part of the odx gene into a pDZ vector (Korean Patent No. 0924065) which is not replicable in *Corynebacterium glutamicum,* and transformation of the recombinant vector into a microorganism and homologous recombination of the recombinant vector into the chromosome were allowed to prepare the microorganism of the genus Corynebacterium with enhanced L-lysine production, in which oxaloacetate decarboxylase is inactivated, but the present application is not limited thereto.

All the *Corynebacterium glutamicum* strains prepared in an embodiment of the present application showed increased L-lysine production, compared to the parent strain, indicating that they are able to produce L-lysine with a high yield by inactivation of the odx gene.

Another aspect of the present application provides a method for producing L-lysine by culturing the microorganism of the genus *Corynebacterium.* In more detail, provided is a method for producing L-lysine, the method including producing L-lysine in a culture or cells of the microorganism by culturing the microorganism of the present application; and recovering L-lysine from the culture or cells of the microorganism, wherein the microorganism is of the genus *Corynebacterium* in which the L-lysine biosynthesis pathway is enhanced and oxaloacetate decarboxylase having an amino acid sequence set forth in SEQ ID NO:1 is inactivated, and wherein the microorganism is a microorganism defined in the claims.

In the method of the present application, the culturing of the microorganism of the genus *Corynebacterium* may be performed by using any culture conditions and methods known in the art, but is not limited to the following example.

A medium which may be used for the culturing of the microorganism of the genus *Corynebacterium* may be exemplified by media disclosed in Manual of Methods for General Bacteriology by the American Society for Bacteriology (Washington D.C., USA, 1981).

Carbon sources to be used in the medium may include saccharides and carbohydrates such as glucose, sucrose, lactose, fructose, maltose, starch and cellulose, oils and lipids such as soybean oil, sunflower seed oil, castor oil and coconut oil, fatty acids such as palmitic acid, stearic acid, and linoleic acid, alcohols such as glycerol and ethanol, and organic acids such as acetic acid. These materials may be used separately or in a mixture.

Nitrogen sources to be used may include peptone, yeast extract, meat broth, malt extract, corn steep liquor, soybean meal and urea, or inorganic compounds, for example, ammonium sulfate, ammonium chloride, ammonium phosphate, ammonium carbonate and ammonium nitrate. These nitrogen sources may be also used separately or in a mixture.

Phosphorus sources to be used may include dipotassium hydrogen phosphate, potassium dihydrogen phosphate, or corresponding sodium-containing salts, and the culture medium may include metal salts such as magnesium sulfate or iron sulfate required for the growth. In addition to these materials, essential growth materials such as amino acids and vitamins may be used. Further, proper precursors may be added to the culture medium. These materials may be added to the culture during cultivation by an appropriate method in a batch or continuous mode.

During the culturing of the microorganism, pH of the culture may be adjusted with a basic compound such as sodium hydroxide, potassium hydroxide or ammonia, or an acidic compound such as phosphoric acid or sulfuric acid. The generation of foam may be restrained using an anti-foaming agent such as fatty acid polyglycol ester. To maintain aerobic conditions, oxygen or an oxygen-containing gas (e.g., air) may be introduced into the culture. The culture temperature may be generally between 20°C and 45°C, and specifically, between 25°C and 40°C. The culturing may be continued until a desired amount of L-lysine is produced. Specifically, the culturing time may be 10 hrs to 160 hrs.

In the method of the present application, the culturing may be performed in a continuous or batch mode such as a batch process, a fed-batch process, and a repeated fed-batch process. The culturing method is well known in the art, and any method may be used.

Hereinafter, the present application will be described in more detail with reference to Examples. However, these Examples are for illustrative purposes only, and the scope of the present application is not intended to be limited by these Examples.

### [Example]

### Example 1: Construction of Recombinant Vector for Inactivation of Corynebacterium-derived odx gene

In order to inactivate an *odx* gene (SEQ ID NO: 2) encoding oxaloacetate decarboxylase of *Corynebacterium glutamicum* having an amino acid sequence represented by SEQ ID NO: 1 by site-specific gene disruption, a plasmid vector was constructed by the following method. First, based on NIH Genbank (USA), a nucleotide sequence of *odx* gene was obtained, and on the basis of this sequence, primers for the preparation of inactivated fragments of the *odx* gene were synthesized.

PCR[Sambrook et al, Molecular Cloning, a Laboratory Manual (1989), Cold Spring Harbor Laboratories] was performed using the chromosome of the wild-type *Corynebacterium glutamicum* ATCC13032 as a template and primers of SEQ ID NO: 3 and SEQ ID NO: 4, SEQ ID NO: 5 and SEQ ID NO: 6. After denaturation at 95°C for 5 minutes, 30 cycles of denaturation at 95°C for 30 seconds, annealing at 55°C for 30 seconds, and polymerization at 72°C for 30 seconds were repeated, and then polymerization at 72°C for 7 minutes was performed. As a result, SEQ ID NO: 7 of 536 bp and SEQ ID NO: 8 of 534 bp including the 5'-end and the 3'-end of the *odx* gene, respectively were obtained.
SEQ ID NO: 3: 5'-TCTAGAACGATGAAATCGCCGCGGGT-3'
SEQ ID NO: 4: 5'-GGGTTGCCCAGCTTGCCGATCACGGGCGGTGAGGTTAGCT-3'
SEQ ID NO: 5: 5'-AGCTAACCTCACCGCCCGTGATCGGCAAGCTGGGCAACCC-3'
SEQ ID NO: 6: 5'-TCTAGAGGTTGCTGCGGATTCTGATT-3'

PCR was performed using the amplified SEQ ID NO: 7 and SEQ ID NO: 8 as a template and SEQ ID NO: 3 and SEQ ID NO: 6. After denaturation at 95°C for 5 minutes, 30 cycles of denaturation at 95°C for 30 seconds, annealing at 55°C for 30 seconds, and polymerization at 72°C for 60 seconds were repeated, and then polymerization at 72°C for 7 minutes was performed. As a result, SEQ ID NO: 9 (hereinbelow, referred to as Δ*odx*) of 1030 bp, in which the 5'-end and the 3'-end of the *odx* gene were linked to each other, was amplified.

A pDZ vector (Korean Patent No. 0924065) which is not replicable in *Corynebacterium glutamicum* and the amplified DNA fragment Δ*odx* were treated with a restriction enzyme XbaI, respectively and then, ligated with each other using DNA ligase. Cloning was performed to obtain a plasmid. This plasmid was designated as pDZ-Δ*odx* (FIG. 1).

### Example 2: Inactivation of odx in L-lysine-Producing Corynebacterium and Analysis of L-lysine Production

*pDZ-Δodx* prepared in Example 1 was transformed into L-lysine-producing strains, *Corynebacterium glutamicum* KCCM11016P and KCCM11347P by an electric pulse method (Appl. Microbiol. Biotechnol.(1999) 52:541-545), respectively and transformed strains were obtained on a selection medium containing 25 mg/L kanamycin. Strains having inactivated *odx* gene were obtained by the DNA fragment Δ*odx* inserted into the genome by secondary recombination (cross-over), and the strains were designated as KCCM11016P/Δ*odx* and KCCM11347P/Δ*odx*, respectively.

In order to examine whether inactivation of the *odx* gene actually influences an increase in lysine production, the *odx*-inactivated strains, KCCM11016P/Δ*odx* and KCCM11347P/Δ*odx* were cultured by the following method and their production was examined.

Each of the strains was inoculated in a 250 ml corner-baffled flask containing 25 ml of the following seed medium, followed by culturing at 37°C for 20 hours under stirring at 200 rpm. 1 mL of the seed culture was inoculated in a 250 ml corner-baffled flask containing 24 ml of the following production medium, followed by culturing at 30°C for 72 hours under stirring at 200 rpm.

### * Composition of seed medium (pH 7.0):

20 g of glucose, 10 g of peptone, 10 g of yeast extract, 5 g of urea, 4 g of KH₂PO₄, 8 g of K₂HPO₄, 0.5 g of MgSO₄·7H₂O, 100 µg of biotin, 1000 µg of thiamine HCl (in 1 liter of process water)

### * Composition of production medium (pH 7.0):

80 g of glucose, 20 g of molasse or pretreated molasse (as reducing sugar), 5 g of corn steep liquor, 40 g of (NH₄)₂SO₄, 4 g of urea, 1 g of KH₂PO₄, 2.5 g of NaCl, 1 g of MgSO₄·7H₂O, 10 mg of FeSO₄·7H₂O, 10 mg of MnSO₄·5H₂O, 100 µg of biotin, 200 µg of thiamine HCl, 40 g of CaCO₃, if necessary, 0.4 g of L-leucine, if necessary, 0.1 g of L-threonine, if necessary, 0.1 g of L-methionine (in 1 liter of process water)

After completion of the culturing, L-lysine production was measured by HPLC. As a result, the content of L-lysine in the culture of each strain was as in the following Table 1.

**[Table 1]**

| | Strain | L-lysine (g/l) | | | |
|---|---|---|---|---|---|
| | | Batch 1 | Batch 2 | Batch 3 | Mean |
| Control group | KCCM11016P | 43.1 | 43.0 | 43.0 | 43.0 |
| Experimental group | KCCM11016P/Δodx | 44.8 | 44.8 | 44.9 | 44.8 |
| Control group | KCCM11347P | 38.1 | 38.1 | 37.9 | 38.0 |
| Experimental group | KCCM11347P/Δodx | 39.5 | 39.6 | 39.6 | 39.6 |

As shown in Table 1, *odx*-inactivated strains, KCCM11016P/Δodx and KCCM11347P/Δodx showed about 4.2% increase in L-lysine production, compared to their parent strains, KCCM11016P and KCCM11347P, respectively.

Of them, the strain KCCM11016P/Δodx was designated as CA01-2276, and deposited in the international depository authority, Korean Culture Center of Microorganisms, which is the Subsidiary Culture Collection of the Korean Federation of Culture Collections located at 361-221, Hongje-1-Dong, Seodaemungu-Gu, Seoul, Korea, on Nov. 22, 2013 under Accession No. KCCM11478P.

### Example 3: Inactivation of odx in L-lysine Biosynthetic Pathway-Enhanced, L-lysine-Producing Corynebacterium and Analysis of lysine Production

pDZ-Δ*odx* prepared in Example 1 was transformed into an L-lysine biosynthetic pathway-enhanced, L-lysine-producing strain, *Corynebacterium glutamicum* KCCM10770P (Korean Patent No. 10-0924065) in the same manner as in Example 2 to prepare a transformed strain, which was designated as KCCM10770P/Δodx.

In order to examine whether inactivation of the *odx* gene actually influences an increase in L-lysine production, the *odx*-inactivated strain, KCCM10770P/Δodx was cultured in the same manner as in Example 2, and its production was examined. The content of L-lysine in the culture of each strain was as in the following Table 2.

**[Table 2]**

| | Strain | L-lysine (g/l) | | | |
|---|---|---|---|---|---|
| | | Batch 1 | Batch 2 | Batch 3 | Mean |
| Control group | KCCM10770P | 47.0 | 47.1 | 46.9 | 47.0 |
| Experimental group | KCCM10770P/Δodx | 50.1 | 50.0 | 50.0 | 50.0 |

As shown in Table 2, the *odx*-inactivated strain, KCCM10770P/Δodx showed about 6.4% increase in L-lysine production, compared to the parent strain, KCCM10770P.

### Example 4: Inactivation of odx in Wild-Type-derived L-lysine-producing Corynebacterium and Analysis of lysine Production

pDZ-Δ*odx* prepared in Example 1 was transformed into a wild-type-derived L-lysine-producing strain, *Corynebacterium glutamicum* CJ3P (Binder et al. Genome Biology 2012, 13:R40, pyc(P458S), hom(V59A), lysC(T311I)) in the same manner as in Example 2 to prepare a transformed strain, which was designated as CJ3P/Δodx.

In order to examine whether inactivation of the *odx* gene actually influences an increase in lysine production, the *odx*-inactivated strain, CJ3P/Δodx was cultured in the same manner as in Example 2, and its production was examined. The content of L-lysine in the culture of each strain was as in the following Table 3.

**[Table 3]**

| | Strain | L-lysine (g/l) | | | |
|---|---|---|---|---|---|
| | | Batch 1 | Batch 2 | Batch 3 | Mean |
| Control group | CJ3P | 7.9 | 8.0 | 8.0 | 8.0 |
| Experimental group | CJ3P/Δodx | 8.2 | 8.3 | 8.3 | 8.3 |

As shown in Table 3, when *odx* was inactivated in the parent strain CJ3P which is the wild-type-derived L-lysine producing strain, about 3.8% increase in the lysine production was also observed.

Accordingly, the present applicants demonstrated in Examples 2 to 4 that increased lysine production was commonly observed in different classes of microorganisms of the genus *Corynebacterium* by inactivation of the *odx* gene, indicating that inactivation of the *odx* gene is an effective trait in the production of L-lysine.
## Claims

1. A microorganism of the genus *Corynebacterium* producing L-lysine, wherein the L-lysine biosynthesis pathway is enhanced and oxaloacetate decarboxylase having an amino acid sequence set forth in SEQ ID NO: 1 is inactivated, wherein the microorganism has an enhanced L-lysine productivity compared to an unmodified microorganism, and wherein the microorganism comprises the *aspB* gene, *lysC* gene, *asd* gene, *dapA* gene, *dapB* gene and *lysA* gene.

2. The microorganism of the genus *Corynebacterium* according to claim 1, wherein the microorganism of the genus *Corynebacterium* is *Corynebacterium glutamicum.*

3. The microorganism of the genus *Corynebacterium* according to claim 1 or 2, wherein the microorganism comprises a deletion or disruption mutation of the gene encoding the oxaloacetate decarboxylase.

4. A method of producing a microorganism of the genus *Corynebacterium* with enhanced L-lysine productivity compared to an unmodified microorganism, the method comprising
providing a microorganism of the genus *Corynebacterium* in which the L-lysine biosynthesis pathway is enhanced and
oxaloacetate decarboxylase having an amino acid sequence set forth in SEQ ID NO:1 is inactivated,
wherein the microorganism of the genus *Corynebacterium* in which the L-lysine biosynthesis pathway is enhanced comprises the *aspB* gene, *lysC* gene, *asd* gene, *dapA* gene, *dapB* gene and *lysA* gene.

5. The method according to claim 4, wherein the microorganism of the genus *Corynebacterium* is *Corynebacterium glutamicum.*

6. The method according to claim 4 or 5, wherein the method comprises inactivating oxaloacetate decarboxylase having an amino acid sequence set forth in SEQ ID NO:1, and the inactivating of oxaloacetate decarboxylase comprises a deletion or disruption mutation of the gene encoding the oxaloacetate decarboxylase.

7. The method according to any one of claims 4-6, wherein the unmodified microorganism is a microorganism of strain KCCM10770P.

8. A microorganism of the genus *Corynebacterium* obtainable by the method of any one of claims 4-7.

9. A method for producing L-lysine, the method comprising:
culturing a microorganism to obtain a culture or cells of the microorganism; and
recovering L-lysine from the culture or cells of the microorganism,
wherein the microorganism is of the genus *Corynebacterium* in which the L-lysine biosynthesis pathway is enhanced and
oxaloacetate decarboxylase having an amino acid sequence set forth in SEQ ID NO:1 is inactivated,
wherein the microorganism is the microorganism of any one of claims 1-3 and 8.

## Patentansprüche

1. Mikroorganismus der Gattung *Corynebacterium,* das L-Lysin herstellt, wobei der L-Lysin-Biosyntheseweg verstärkt ist und Oxalacetatdecarboxylase mit einer in SEQ ID NO: 1 dargestellten Aminosäuresequenz inaktiviert wird, wobei der Mikroorganismus eine erhöhte L-Lysinproduktivität verglichen mit einem unmodifizierten Mikroorganismus aufweist, wobei der Mikroorganismus das *aspB*-Gen, das *lysC*-Gen, das *asd*-Gen, das *dapA*-Gen, das *dapB*-Gen und das *lysA*-Gen umfasst.

2. Mikroorganismus der Gattung *Corynebacterium* nach Anspruch 1, wobei der Mikroorganismus der Gattung *Corynebacterium Corynebacterium glutamicum ist.*

3. Mikroorganismus der Gattung *Corynebacterium* nach Anspruch 1 oder 2, wobei der Mikroorganismus eine Deletions- oder Störungssmutation des Gens, das das Oxalacetat Decarboxylase kodiert, umfasst.

4. Verfahren zur Herstellung eines Mikroorganismus der Gattung *Corynebacterium* mit einer verstärkten L-Lysin-Produktivität verglichen mit einem unmodifizierten Mikroorganismus, wobei das Verfahren das Bereitstellen eines Mikroorganismus der Gattung *Corynebacterium* , in dem der L-Lysin-Biosyntheseweg verstärkt ist, umfasst und Oxalacetatdecarboxylase mit einer in der SEQ ID NO:1 dargestellten Aminosäuresequenz inaktiviert wird, wobei der Mikroorganismus der Gattung *Corynebacterium,* in dem der L-Lysin-Biosyntheseweg verstärkt ist, das *aspB-* Gen, das *lysC*-Gen, das *asd*-Gen, das *dapA*-Gen, das *dapB*-Gen und das *lysA*-Gen umfasst.

5. Verfahren nach Anspruch 4, wobei der Mikroorganismus der Gattung *Corynebacterium Corynebacterium glutamicum* ist.

6. Verfahren nach Anspruch 4 oder 5, wobei das Verfahren das Inaktivieren von Oxalacetat Decarboxylase mit einer in SEQ ID NO: 1 dargestellten Aminosäuresequenz umfasst, und das Inaktivieren der Oxalacetat Decarboxylase eine Deletions- oder Störungssmutation des Gens umfasst, das das Oxaloacetat Decarboxylase kodiert, umfasst.

7. Verfahren nach einem der Ansprüche 4 bis 6, wobei der unmodifizierte Mikroorganismus ein Mikroorganismus des Stammes KCCM10770P ist.

8. Mikroorganismus der Gattung *Corynebacterium,* erhältlich durch das Verfahren nach einem der Ansprüche 4-7.

9. Verfahren zum Herstellen von L-Lysin, wobei das Verfahren Folgendes umfasst:
Kultivieren eines Mikroorganismus, um eine Kultur oder Zellen des Mikroorganismus zu erhalten; und
Zurückgewinnen von L-Lysin aus der Kultur oder den Zellen des Mikroorganismus, wobei der Mikroorganismus aus der Gattung *Corynebacterium* ist, in welchem der L-Lysin-Biosyntheseweg verstärkt ist und die Oxaloacetat Decarboxylase mit einer in SEQ ID NO: 1 dargestellten Aminosäuresequenz inaktiviert ist, wobei der Mikroorganismus der Mikroorganismus nach einem der Ansprüche 1-3 und 8 ist.

## Revendications

1. Microorganisme du genre *Corynebacterium* produisant de la L-lysine, la voie de biosynthèse de la L-lysine étant améliorée et l'oxaloacétate décarboxylase ayant une séquence d'acides aminés présentée dans SEQ ID N° : 1 étant inactivée, le microorganisme ayant une productivité de L-lysine améliorée comparé à un microorganisme non modifié, et le microorganisme comprenant le gène *aspB,* le gène *lysC,* le gène *asd,* le gène *dapA,* le gène *dapB* gène et le gène *lysA.*

2. Microorganisme du genre *Corynebacterium* selon la revendication 1, dans lequel le microorganisme du genre *Corynebacterium* est le *Corynebacterium glutamicum.*

3. Microorganisme du genre *Corynebacterium* selon la revendication 1 ou 2, dans lequel le microorganisme comprend une mutation par délétion ou perturbation du gène codant pour l'oxaloacétate décarboxylase.

4. Procédé de production d'un microorganisme du genre *Corynebacterium* doté d'une productivité de L-lysine améliorée comparé à un microorganisme non modifié, le procédé comprenant la fourniture d'un microorganisme du genre *Corynebacterium* dans lequel la voie de biosynthèse de la L-lysine est améliorée et l'oxaloacétate décarboxylase ayant un séquence d'acides aminés présentée dans SEQ ID N° : 1 étant inactivé, le microorganisme du genre *Corynebacterium* dans lequel la voie de biosynthèse de la l-lysine est améliorée comprenant le gène *aspB,* le gène *lysC,* le gène *asd,* le gène *dapA,* le gène *dapB* et le gène *lysA.*

5. Procédé selon la revendication 4, dans lequel le microorganisme du genre *Corynebacterium* est le *Corynebacterium glutamicum.*

6. Procédé selon la revendication 4 ou 5, dans lequel le procédé comprend l'inactivation de l'oxaloacétate décarboxylase ayant une séquence d'acides aminés présentée dans SEQ ID N° : 1, et l'inactivation de l'oxaloacétate décarboxylase comprend une mutation par délétion ou perturbation du gène codant pour l'oxaloacétate décarboxylase.

7. Procédé selon l'une quelconque des revendications 4 à 6, dans lequel le microorganisme non modifié est un microorganisme de souche KCCM10770P.

8. Microorganisme du genre *Corynebacterium* pouvant être obtenu par le procédé selon l'une quelconque des revendications 4 à 7.

9. Procédé de production de L-lysine, le procédé comprenant :
la culture d'un microorganisme pour obtenir une culture ou des cellules du microorganisme ; et
la récupération de L-lysine de la culture ou des cellules du microorganisme, le microorganisme appartenant au genre *Corynebacterium* dans lequel la voie de biosynthèse de la L-lysine est améliorée et l'oxaloacétate décarboxylase ayant une séquence d'acides aminés présentée dans SEQ ID N° : 1 étant inactivée, le microorganisme étant le microorganisme selon l'une quelconque des revendications 1 à 3 et 8.


<110> CJ CheilJedang Corporation
<120> A microorganism of corynebacterium genus having enhanced L-lysine productivity and method for producing L-lysine using the same
<130> PX048854PCT
<150> KR 14/057,966
   <151> 2014-05-14
<160> 9
<170> KopatentIn 2.0
<210> 1
   <211> 268
   <212> PRT
   <213> Corynebacterium glutamicum
<400> 1
   Met Arg Phe Gly Arg Ile Ala Thr Pro Asp Gly Met Cys Phe Cys Ser
   1               5                   10                  15
   Ile Glu Gly Glu Gly Asp Asp Val Ala Asn Leu Thr Ala Arg Glu Ile
               20                  25                  30
   Glu Gly Thr Pro Phe Thr Glu Pro Lys Phe Thr Gly Arg Glu Trp Pro
           35                  40                  45
   Leu Lys Asp Val Arg Leu Leu Ala Pro Met Leu Pro Ser Lys Val Val
       50                  55                  60
   Ala Ile Gly Arg Asn Tyr Ala Asp His Val Ala Glu Val Phe Lys Lys
   65                  70                  75                  80
   Ser Ala Glu Ser Leu Pro Pro Thr Leu Phe Leu Lys Pro Pro Thr Ala
                   85                  90                  95
   Val Thr Gly Pro Glu Ser Pro Ile Arg Ile Pro Ser Phe Ala Thr Lys
               100                 105                 110
   Val Glu Phe Glu Gly Glu Leu Ala Val Val Ile Gly Lys Pro Cys Lys
           115                 120                 125
   Asn Val Lys Ala Asp Asp Trp Lys Ser Val Val Leu Gly Phe Thr Ile
       130                 135                 140
   Ile Asn Asp Val Ser Ser Arg Asp Leu Gln Phe Ala Asp Gly Gln Trp
   145                 150                 155                 160
   Ala Arg Ala Lys Gly Ile Asp Thr Phe Gly Pro Ile Gly Pro Trp Ile
                   165                 170                 175
   Glu Thr Asp Ile Asn Ser Ile Asp Leu Asp Asn Leu Pro Ile Lys Ala
               180                 185                 190
   Arg Leu Thr His Asp Gly Glu Thr Gln Leu Lys Gln Asp Ser Asn Ser
           195                 200                 205
   Asn Gln Met Ile Met Lys Met Gly Glu Ile Ile Glu Phe Ile Thr Ala
       210                 215                 220
   Ser Met Thr Leu Leu Pro Gly Asp Val Ile Ala Thr Gly Ser Pro Ala
   225                 230                 235                 240
   Gly Thr Glu Ala Met Val Asp Gly Asp Tyr Ile Glu Ile Glu Ile Pro
                   245                 250                 255
   Gly Ile Gly Lys Leu Gly Asn Pro Val Val Asp Ala
               260                 265
<210> 2
   <211> 807
   <212> DNA
   <213> Corynebacterium glutamicum
<400> 2
   atgcgttttg gacgaattgc caccccagat ggaatgtgtt tttgctcaat tgaaggcgaa      60
   ggcgatgatg tagctaacct caccgcccgt gagatcgagg gtaccccctt cactgaacca     120
   aagttcaccg gtcgcgaatg gccacttaaa gatgtacgcc tgctggcacc aatgctgcca     180
   agcaaagtcg tcgcgattgg ccgtaactac gccgatcacg ttgctgaggt gtttaagaag     240
   tccgcagaat cactgccacc aaccctgttc ctgaagccac caacagctgt caccggacca     300
   gagtccccaa tccgaatccc ttcctttgcc accaaggtgg aattcgaagg tgagctcgca     360
   gtagttatcg gcaagccctg caagaacgtc aaggctgatg actggaagtc tgtcgttttg     420
   ggcttcacca tcatcaacga cgtctcctcc cgtgacctcc agttcgctga cggccagtgg     480
   gcacgcgcta agggcattga caccttcggc cccatcggac catggattga aactgacatc     540
   aactccatcg acttggacaa cctgcccatc aaggcacgcc tcacccacga cggcgaaacc     600
   caattgaagc aggactccaa ctccaaccag atgatcatga agatgggcga aattatcgag     660
   ttcatcaccg cctccatgac cctgctccca ggcgacgtta ttgcaaccgg ttctccagca     720
   ggcaccgaag caatggttga cggcgactac atcgaaatcg aaattccagg catcggcaag     780
   ctgggcaacc cagttgtgga cgcctaa                                         807
<210> 3
   <211> 26
   <212> DNA
   <213> Artificial Sequence
<220>
   <223> primer
<400> 3
   tctagaacga tgaaatcgcc gcgggt 26
<210> 4
   <211> 40
   <212> DNA
   <213> Artificial Sequence
<220>
   <223> primer
<400> 4
   gggttgccca gcttgccgat cacgggcggt gaggttagct 40
<210> 5
   <211> 40
   <212> DNA
   <213> Artificial Sequence
<220>
   <223> primer
<400> 5
   agctaacctc accgcccgtg atcggcaagc tgggcaaccc 40
<210> 6
   <211> 26
   <212> DNA
   <213> Artificial Sequence
<220>
   <223> primer
<400> 6
   tctagaggtt gctgcggatt ctgatt 26
<210> 7
   <211> 536
   <212> DNA
   <213> Artificial Sequence
<220>
   <223> odx 5' terminal
<400> 7
   tctagaacga tgaaatcgcc gcgggtatcg acatcaaaga ggcgcttggc caactactgc      60
   aggcgctcca gggacgcgcc atgctggcgc atttttcacc catcgagcgc gattttattt     120
   cggccgcatg cctcaagcat ttcggaacgc ttctcgacgt ccccctcgtg gatacttttg    180
   ccatggaacg ccgccacatg gagaggatgt ccacctaccc acgtggcgaa gacctccggc     240
   ttgcacgcat tcgacaacga tatggtcttc ccaattactc caatcaccag gcattaaccg     300
   acgctttggc gtgcgccgag gtgtatttgg tgcagatcac gcaccttcgg gcgaacaccc     360
   tcaaagatat ttgggaatag gggtcatcgt gcaggccccc acacccaacg cgtaacataa     420
   cggtcatgcg ttttggacga attgccaccc cagatggaat gtgtttttgc tcaattgaag     480
   gcgaaggcga tgatgtagct aacctcaccg cccgtgatcg gcaagctggg caaccc         536
<210> 8
   <211> 534
   <212> DNA
   <213> Artificial Sequence
<220>
   <223> odx 3' terminal
<400> 8
   agctaacctc accgcccgtg atcggcaagc tgggcaaccc agttgtggac gcctaaaatg 60
   gatcaccacc atcacgatca tggggagaag cgtccgtacg accaggtcac cccagaatcc 120
   atggagaatc aatacaccac cacggaacaa gtctggagcg gcaaccccaa ccaagcactc 180
   atcgcctact tgggtgaaga gccagctgaa ggcaccgcac tagacattgg ctgtggcgaa 240
   ggcgctgacc tcgtgtggct gtcctccaag ggatacgtca ccaccggcat cgacttcgca 300
   ccaactgcag tgaagcgcgc gaaacaaaac gccccatcag ccgaagtcct cttggtatca 360
   ttcaccgaat tcgccatcga atccggtcgc caattcgacc tcgtcagctg ctcgtacggc 420
   caaatcccag ccaacgacac ctccgtcgcg caactggaaa acctcgtcgc acccggcgga 480
   accctcctgt tcacccacca cgactttgaa tcagaatccg cagcaacctc taga 534
<210> 9
   <211> 1030
   <212> DNA
   <213> Artificial Sequence
<220>
   <223> odx
<400> 9
   tctagaacga tgaaatcgcc gcgggtatcg acatcaaaga ggcgcttggc caactactgc 60
   aggcgctcca gggacgcgcc atgctggcgc atttttcacc catcgagcgc gattttattt 120
   cggccgcatg cctcaagcat ttcggaacgc ttctcgacgt ccccctcgtg gatacttttg 180
   ccatggaacg ccgccacatg gagaggatgt ccacctaccc acgtggcgaa gacctccggc 240
   ttgcacgcat tcgacaacga tatggtcttc ccaattactc caatcaccag gcattaaccg 300
   acgctttggc gtgcgccgag gtgtatttgg tgcagatcac gcaccttcgg gcgaacaccc 360
   tcaaagatat ttgggaatag gggtcatcgt gcaggccccc acacccaacg cgtaacataa 420
   cggtcatgcg ttttggacga attgccaccc cagatggaat gtgtttttgc tcaattgaag 480
   gcgaaggcga tgatgtagct aacctcaccg cccgtgatcg gcaagctggg caacccagtt 540
   gtggacgcct aaaatggatc accaccatca cgatcatggg gagaagcgtc cgtacgacca 600
   ggtcacccca gaatccatgg agaatcaata caccaccacg gaacaagtct ggagcggcaa 660
   ccccaaccaa gcactcatcg cctacttggg tgaagagcca gctgaaggca ccgcactaga 720
   cattggctgt ggcgaaggcg ctgacctcgt gtggctgtcc tccaagggat acgtcaccac 780
   cggcatcgac ttcgcaccaa ctgcagtgaa gcgcgcgaaa caaaacgccc catcagccga    840
   agtcctcttg gtatcattca ccgaattcgc catcgaatcc ggtcgccaat tcgacctcgt    900
   cagctgctcg tacggccaaa tcccagccaa cgacacctcc gtcgcgcaac tggaaaacct    960
   cgtcgcaccc ggcggaaccc tcctgttcac ccaccacgac tttgaatcag aatccgcagc   1020
   aacctctaga                                                          1030